# EUROPEAN PATENT APPLICATION

(11) **EP 3 815 740 A1**
(43) Date of publication of application: **05.05.2021**
(21) Application number: 19824894.0
(22) Date of filing: 28.06.2019
(51) Int. Cl.: A61N 1/44, A61B 18/04, A61B 90/50, A61C 1/08, B01J 19/08, H05H 1/24

(54) **PLASMA IRRADIATION APPARATUS**

(30) Priority: 29.06.2018 JP 2018124456
(71) Applicant: SEKISUI CHEMICAL CO., LTD., Osaka-shi Osaka 530-8565 (JP)
(72) Inventor: TAKATA, Masahiro, Ibaraki 300-4292 (JP); UEHARA, Tsuyoshi, Kyoto 601-8105 (JP); TOGI Akiko, Ibaraki 300-4292 (JP); OSHITA, Takaya, Ibaraki 300-4292 (JP); ATAKA, Motoharu, Ibaraki 300-4292 (JP); TADA, Mayuka, Ibaraki 300-4292 (JP); NAGAHARA, Yu, Kyoto 601-8105 (JP); INOUE, Tsuyoshi, Kyoto 601-8105 (JP)
(74) Representative: Ter Meer Steinmeister & Partner
(86) International application number: PCT/JP2019/025929
(87) International publication number: WO 2020/004650

(57) **Abstract**

A plasma application apparatus including: an application instrument which has a plasma generation unit, and is configured to discharge at least one of plasma generated by the plasma generation unit and a reactive gas generated by the plasma; and a supply unit for supplying electric power and a plasma generating gas to the application instrument, a supporting part extending upward from the supply unit, a power/gas supply line connecting the application instrument to the supply unit, and a connecting part connecting at least one of the application instrument and the power/gas supply line to the supporting part,

## Description

### TECHNICAL FIELD

The present invention relates to a plasma application apparatus.

### BACKGROUND ART

Conventionally, a plasma application apparatus for medical use such as dental treatment has been known.

The plasma application apparatus cures the affected area by applying plasma or reactive gas to the affected area such as wounds. The reactive gas is generated by plasma in a plasma application apparatus.

For example, Patent Document 1 discloses a plasma jet application apparatus for implementing dental treatment. The plasma jet application apparatus is equipped with an application instrument having a plasma jet application means. The plasma jet application apparatus generates plasma and applies the generated plasma together with reactive species to a target object. The reactive species are generated by reaction of the plasma with the gas present within or around the plasma.

Patent Document 2 discloses a plasma application apparatus that generates reactive gas (reactive species) inside an application instrument, and discharges the reactive gas from the nozzle of the application instrument to apply the reactive gas to an affected area of a patient. The reactive gas is, for example, active oxygen or active nitrogen.

### Description of Prior Art

### Patent Document

Patent Document 1: Japanese Patent Granted Publication No. 5441066
Patent Document 2: Japanese Unexamined Patent Application Publication No. 2017-50267

### DISCLOSURE OF INVENTION

### Problems to be Solved by the Invention

However, with this type of plasma application apparatus, the user may accidentally drop the application instrument on the floor or the like during treatment. This is not desirable because the dropped application instrument may suffer damage or failure. On the other hand, if the movable range of the application instrument is overly restricted to prevent such accidental fall of the application instrument, such restricted movable range may in turn lead to inferior operability of the application instrument. Therefore, it has been desired to make an improvement to prevent the accidental fall of the application instrument while ensuring the operability of the application instrument.

The present invention has been made in view of these problems, and the purpose of the present invention is to provide a plasma application apparatus that prevents the user from accidentally dropping the application instrument during use, while ensuring the operability of the application instrument.

### Means to Solve the Problems

Embodiments proposed by the present invention in order to solve the above-mentioned problems are as enumerated below.

The plasma application apparatus of the present invention includes: an application instrument which has a plasma generation unit, and is configured to discharge at least one of plasma generated by the plasma generation unit and a reactive gas generated by the plasma; a supply unit for supplying electric power and a plasma generating gas to the application instrument; a supporting part extending upward from the supply unit; a power/gas supply line connecting the application instrument to the supply unit; and a connecting part connecting at least one of the application instrument and the power/gas supply line to the supporting part, wherein the supporting part and the connecting part maintain the application instrument to stay at a position above a ground surface on which the supply unit is placed.

According to the present invention, for example, even when an application instrument accidentally drops from the hand of a user holding the application instrument, at least one of the application instrument and the power/gas supply line is fixed to the supporting part through the connecting part. Therefore, even if the user releases the application instrument, the downward movement of the application instrument is restricted, and the user can prevent the application instrument from dropping to the ground during use. In the meantime, since the fixing of the application instrument to the supporting part is implemented via the connecting part, the freedom of movement of the application instrument in a direction other than the downward direction is appropriately ensured when operating the application instrument. Consequently, the operability of the plasma application apparatus is also ensured satisfactorily.

The plasma application apparatus may have a configuration wherein the connecting part is a linear member, one end (A) of the linear member is fixed to at least one of the application instrument and the power/gas supply line, the other end (B) of the linear member is fixed to the supporting part, and the supporting part has a housing section capable of winding in the linear member and unwinding the linear member therefrom.

According to this invention, when the linear member need not be long in relative terms, the linear member can be wound into the housing section, whereas when the linear member needs to be long in relative terms, the linear member can be unwound from the housing section. Thus, the length of a part of the linear member which is withdrawn out of the housing section can be adjusted.

Further, the plasma application apparatus may have a configuration wherein the one end (A) of the linear member is fixed to the application instrument while the other end (B) of the linear member is fixed to the supporting section at its position higher than the one end (A) of the linear member, and the length of the linear member is smaller than the height of the fixed position of the other end (B) from the ground surface.

According to the this invention, even when the application instrument accidentally drops from the hand of a user holding the application instrument, it is possible to prevent the dropped application instrument from coming into contact with the floor surface or the ground (i.e., a ground surface on which the supply unit is installed).

Further, the plasma application apparatus may have a configuration wherein the supporting part comprises a flexible rod, wherein the connecting part is a banding band that fixes the flexible rod and the power/gas supply line, the power/gas supply line and the flexible rod are respectively positioned on an upper surface of the supply unit so as to extend upward, and a middle part of the power/gas supply line is fixed to the flexible rod by the banding band, thereby maintaining the middle part of the power/gas supply line at a position higher than the upper surface of the supply unit.

According to this invention, since the power/gas supply line is provided on the upper surface of the supply unit so as to extend upward, it is possible to suppress damage caused by contact of the power/gas supply line with a wall or the like. In addition, the flexible rod can prevent the user from dropping the application instrument accidentally during use, while ensuring the operability of the reactive gas application apparatus as well as maintaining a simple structure and keeping the entire size of the reactive gas apparatus compact.

The plasma application apparatus may also have a configuration wherein the plasma generating unit generates the plasma by dielectric barrier discharge.

Further, the plasma application apparatus may have a configuration wherein the plasma generating unit generates the plasma by using nitrogen gas.

### Effect of the Invention

The plasma application apparatus of the present invention can prevent the user from accidentally dropping the application instrument during use, while ensuring the operability of the application instrument.

### BRIEF DESCRIPTION OF THE DRAWINGS

FIG. 1 is a schematic view of a reactive gas application apparatus according to the first embodiment of the present invention.
FIG. 2 is block diagram showing a schematic configuration of the reactive gas application apparatus.
FIG. 3 is a longitudinal sectional view of the reactive gas application apparatus.
FIG. 4 is a cross-sectional view showing the application apparatus of FIG. 3 as viewed from the arrow direction of the A1-A1 line of FIG. 3.
Fig. 5 is a schematic view showing a state in which the user using the reactive gas application apparatus has accidentally dropped the application instrument.
FIG. 6 is a schematic view showing the reactive gas application apparatus according to a second embodiment of the present invention.

### DESCRIPTION OF THE EMBODIMENTS

### (First Embodiment)

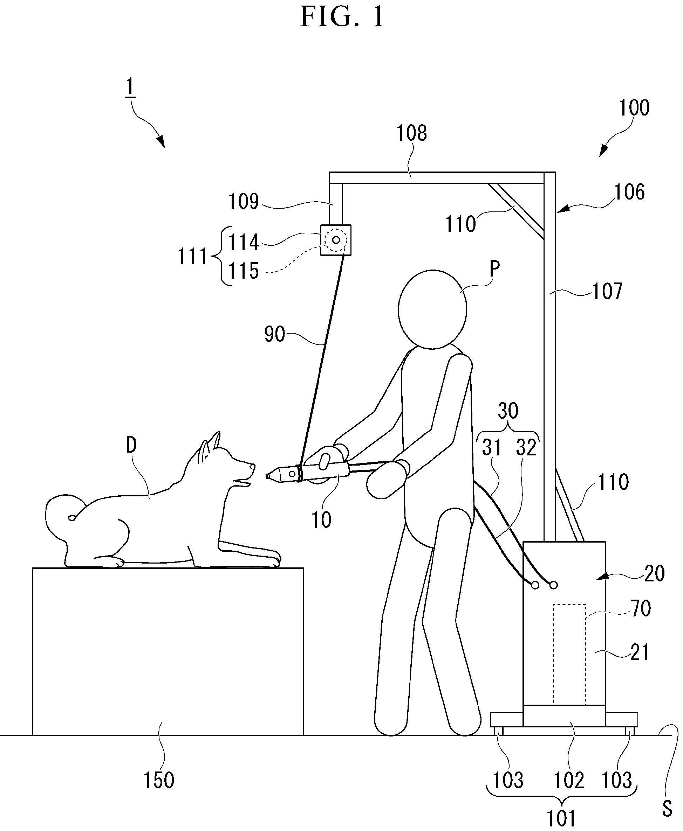

Hereinbelow, the first embodiment of the plasma application apparatus of the present invention is described with reference to FIGs. 1 to 5.

The plasma application apparatus of the present embodiment is a plasma jet application apparatus or a reactive gas application apparatus.

The plasma jet application apparatus generates plasma. The plasma jet application apparatus generates plasma and applies the generated plasma together with reactive species to a target object. The reactive species are generated by reaction of the plasma with the gas present within or around the plasma. Examples of the reactive species include reactive oxygen species and reactive nitrogen species. Examples of the reactive oxygen species include hydroxyl radicals, singlet oxygen, ozone, hydrogen peroxide, and superoxide anion radicals. Examples of the reactive nitrogen species include nitric oxide, nitrogen dioxide, peroxynitrite, peroxynitrite, and dinitrogen trioxide.

On the other hand, the reactive gas application apparatus generates plasma and applies a reactive gas containing reactive species generated by the plasma to a target object. The reactive species are generated by reaction of the plasma with the gas present within or around the plasma.

Hereinbelow, an explanation is made taking, as an example, a case where the plasma application apparatus is a reactive gas application apparatus.

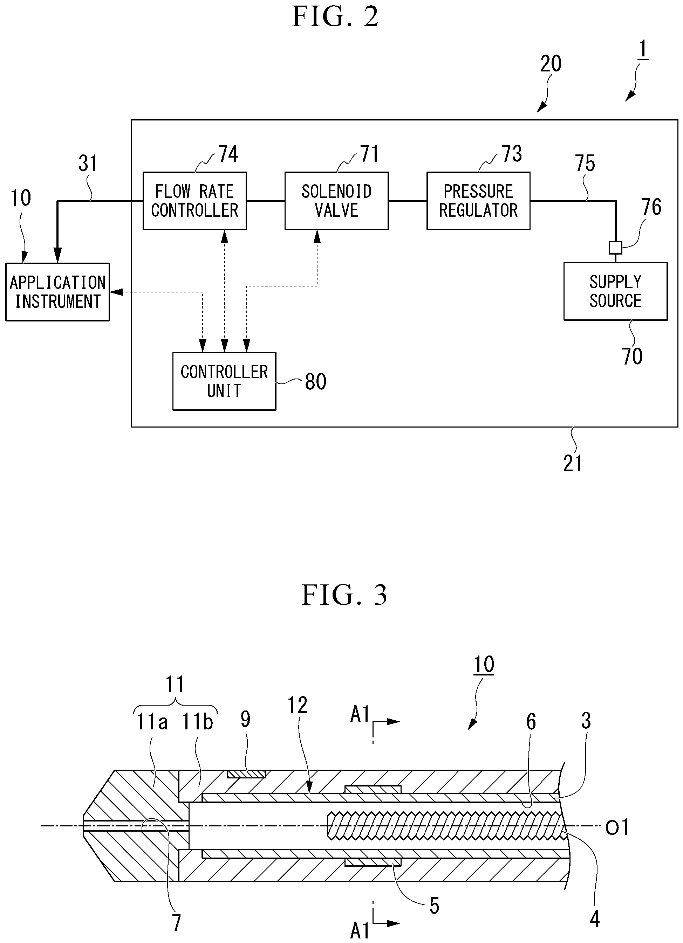

As shown in FIGs. 1 and 2, the reactive gas application apparatus 1 of the present embodiment includes an application instrument 10, a supply unit 20, a power/gas supply line 30, a supply source 70, a control unit 80, and a wire (linear member) 90 and a supporting part 100.

The application instrument 10 discharges the reactive gas generated in the application instrument 10.

The supply unit 20 supplies electric power and plasma generating gas to the application instrument 10. Examples of the plasma generating gas include noble gases such as helium, neon, argon and krypton; nitrogen (nitrogen gas); and the like. With respect to these gases, a single type thereof may be supplied individually or two or more types thereof may be supplied in combination.

The plasma generating gas preferably contains nitrogen gas as a main component. In this context, the nitrogen gas being contained as a main component means that the amount of the nitrogen gas contained in the plasma generating gas is more than 50 % by volume.

The supply unit 20 houses the supply source 70. The supply source 70 contains the plasma generating gas. The supply unit 20 is connected to a power supply (not shown), such as a 100 V household power supply.

The power/gas supply line 30 includes a gas conduit 31 and an electric wiring 32.

The gas conduit 31 connects the application instrument 10 with the supply unit 20. The electrical wiring 32 connects the application instrument 10 with the supply unit 20.

In the present embodiment, the gas conduit 31 and the electric wiring 32 are provided independently from each other, but the gas conduit 31 and the electric wiring 32 may be integrated.

FIG. 3 is a cross-sectional view (longitudinal section) showing a plane along the axis of the application instrument 10.

As shown in FIG. 3, the application instrument 10 includes an elongated cowling 11, and a plasma generating unit 12 provided in the cowling 11.

The cowling 11 includes a cylindrical body 11b and a head 11a covering the tip of the body 11b. The body 11b is not limited to that of a cylindrical shape, and may be of a polygonal tube shape such as a square tube shape, a hexagonal tube shape, an octagonal tube shape or the like.

The head 11a gradually narrows toward the tip thereof. That is, the head 11a in the present embodiment has a conical shape. The head 11a is not limited to that of a conical shape, and may be of a polygonal cone shape such as a quadrangular pyramid shape, a hexagonal pyramid shape, an octagonal pyramid shape or the like.

A reactive gas flow path 7 extending in the tube axis O1 direction is provided inside the head 11a. The tube axis O1 is a tube axis of the body 11b.

The material of the head 11a is not particularly limited, and may or may not be an insulating material. The material of the head 11a is preferably a material excellent in abrasion resistance and corrosion resistance. As an example of such a material excellent in abrasion resistance and corrosion resistance, a metal such as stainless steel can be listed.

The material of the body 11b is not particularly limited, but is preferably an insulating material. The body 2b may be formed of only an electrically insulating material, or may have a multilayer structure having a layer of an electrically insulating material and a layer of a metal material formed on the surface thereof.

Examples of the insulating material include thermoplastic resin, thermosetting resin, etc. Examples of the thermoplastic resin include polyethylene, polypropylene, polyvinyl chloride, polystyrene, acrylonitrile-butadiene-styrene resin (ABS resin), etc. Examples of the thermosetting resin include phenol resin, melamine resin, urea resin, epoxy resin, unsaturated polyester resin, silicon resin, etc.

Examples of the metal material include stainless steel, titanium, aluminum, and the like.

The materials of the body 11b the head 11a may be the same or different.

The size of the body 11b is not particularly limited, and may be such a size that allows the body 11b to be easily grasped with fingers.

The body 11b has an operation switch 9 (operation unit) on its outer peripheral surface. The operation switch 9 may not be provided on the application instrument 10, but may be provided on the supply unit 20 or provided in the form of a foot switch.

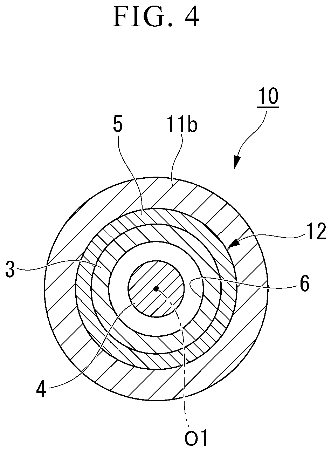

As shown in FIGs. 3 and 4, the plasma generating unit 12 has a tubular dielectric 3 (dielectric), an inner electrode 4, and an outer electrode 5.

The tubular dielectric 3 is a cylindrical member extending in the tube axis O1 direction. The tubular dielectric 3 has in its inside a gas flow path 6 extending in the tube axis O1 direction. The gas flow path 6 communicates with a reactive gas flow path 7. The tube axis O1 coincides with the tube axis of the tubular dielectric 3.

As a material of the tubular dielectric 3, a dielectric material used for a known plasma generator can be employed. Examples of the material of the tubular dielectric 3 include glass, ceramics, synthetic resins, and the like. The dielectric constant of the tubular dielectric 3 is preferably as low as possible.

In the tubular dielectric 3, an inner electrode 4 is provided. The inner electrode 4 is a substantially columnar member extending in the tube axis O1 direction. The inner electrode 4 is spaced apart from the inner surface of the tubular dielectric 3.

The inner electrode 4 includes a shaft portion extending in the tube axis O1 direction and a screw thread on the outer peripheral surface of the shaft portion. The shaft portion may be solid or hollow. Of these, a solid shaft portion is more preferable. The solid shaft portion allows easy processing and improves mechanical durability.

The screw thread of the inner electrode 4 is a helical screw thread that circulates in the circumferential direction of the shaft portion. The shape of the inner electrode 4 is the same as that of a screw or a bolt.
The screw thread on the outer peripheral surface of the inner electrode 4 allows the electric field at the tip of the screw thread to be locally enhanced, thereby lowering the discharge inception voltage. Therefore, plasma can be generated and maintained with less electric power.

The inner electrode 4 may not have concavities and convexities such as screw threads on the outer peripheral surface. That is, the inner electrode 4 may be a cylinder without any concavities or convexities on its outer peripheral surface.

The material of the inner electrode 4 is not particularly limited as long as the material is electrically conductive, and metals used for electrodes of known plasma generating apparatuses can be used. Examples of the material of the inner electrode 4 include metals such as stainless steel, copper and tungsten, carbon, and the like.

On the outer peripheral surface of the tubular dielectric 3, an outer electrode 5 extending along the inner electrode 4 is provided. The outer electrode 5 is an annular electrode that surrounds the outer peripheral surface of the tubular dielectric 3.

The material of the outer electrode 5 is not particularly limited as long as the material is electrically conductive, and metals used for electrodes of known plasma generating apparatuses can be used. Examples of the material of the outer electrode 5 include metals such as stainless steel, copper and tungsten, carbon, and the like.

As shown in FIG. 4, the tubular dielectric 3, the inner electrode 4, and the outer electrode 5 are positioned concentrically around the tube axis O1.

In the present embodiment, the outer peripheral surface of the inner electrode 4 and the inner peripheral surface of the outer electrode 5 face each other through the tubular dielectric 3.

In the present embodiment, the plasma generating unit 12 generates plasma by dielectric barrier discharge.

The plasma generating unit 12 generates plasma using, for example, nitrogen. In order to generate plasma using nitrogen, the application of higher voltage to the plasma generating unit 12 is required, which in turn necessitates the use of a heavier shield against electromagnetic waves emitted from the plasma generating unit 12. Consequently, the accidental fall of the plasma generating unit 12 is more likely to result in breakage of the dielectric for generating the dielectric barrier discharge.

The plasma generating unit 12 can be detached from the cowling 11. For example, the plasma generating unit 12 is configured to be able to be pulled out from the cowling 11 in the direction of the tube axis O1. For example, the plasma generating unit 12 may be configured so that, after the cowling 11 is disassembled into the head 11a and the body 11b, the plasma generating unit 12 is pulled out to the front side relative to the body 11b, with the proviso that the side of the head 11a is regarded as the front side and the side of the body 11b is regarded as the rear side as viewed along the axis O1 direction).

For example, when the plasma generating unit 12 is damaged, a new plasma generating unit 12 can be attached to the cowling 11 after the damaged plasma generating unit 12 has been separated from the cowling 11. In this case, the new plasma generating unit 12 can be inserted into the cowling 11 in the direction of the tube axis O1.

The supply unit 20, as shown in FIG. 1, supplies electricity and plasma generating gas to the application instrument 10. The supply unit 20 is capable of adjusting the voltage and frequency applied between the inner electrode 4 and the outer electrode 5.

The supply unit 20 has a housing 21 that houses the supply source 70. For example, the housing 21 is formed in a rectangular parallelepiped box shape. The housing 21 houses the supply source 70 in a detachable manner. Thus, when the gas in the supply source 70 housed in the housing 21 runs out, the supply source 70 can be replaced.

The supply source 70 supplies the plasma generating gas to the plasma generating unit 12. The supply source 70 is a pressure-resistant vessel filled with the plasma generating gas. As shown in FIG. 2, the supply source 70 is detachably attached to the pipe 75 disposed in the housing 21. The pipe 75 connects the supply source 70 with the gas conduit 31.

A solenoid valve 71, a pressure regulator 73, and a flow rate controller 74 are attached to the pipe 75.

When the solenoid valve 71 is opened, the plasma generating gas is supplied from the supply source 70 to the application instrument 10 through pipe 75 and gas conduit 31. In the example shown in the drawing, the solenoid valve 71 is not configured to enable adjustment of the valve opening degree, but is configured to enable only switch between opening and closing. However, the solenoid valve 71 may also be configured to enable adjustment of the valve opening degree.

The pressure regulator 73 is positioned between the solenoid valve 71 and the supply source 70. The pressure regulator 73 lowers the pressure of the plasma generating gas from the supply source 70 to the solenoid valve 71 (i.e., the pressure regulator 73 reduces the pressure of the plasma generating gas).

The flow rate controller 74 is disposed between the solenoid valve 71 and the gas conduit 31. The flow rate controller 74 adjusts the flow rate (supply rate per unit time) of the plasma generating gas having passed through the solenoid valve 71. For example, the flow rate controller 74 adjusts the flow rate of the plasma generating gas to 3 L/min.

A joint 76 is provided at the end of pipe 75 on the supply source 70-side. The supply source 70 is detachably attached to the joint 76. The attachment or detachment of the supply source 70 to or from the joint 76 allows for replacement of the supply source 70 while leaving the solenoid valve 71, the pressure regulator 73, and the flow rate controller 74 (hereinafter, collectively referred to as "solenoid valve 71, etc.") fixed to the housing 21.

In this case, a common solenoid valve 71, etc. can be used for both the old and new supply sources 70 before and after the replacement. In addition, the solenoid valve 71, etc. may be integrally fixed to the supply source 70 so as to detachable from the housing 21 together with the supply source 70.

As shown in FIG. 1, the gas conduit 31 forms a path for supplying the plasma generating gas from the supply unit 20 to the application instrument 10. The gas conduit 31 is connected to the rear end of the tubular dielectric 3 of the application instrument 10.

The material of the gas conduit 31 is not particularly limited, and a material used for known gas pipes can be used. Concerning a material of the gas conduit 31, a resin pipe, a rubber tube and the like can be listed as examples, and a material having flexibility is preferable.

It is preferable that the gas conduit 31 used in the present invention has excellent flexibility as well as appropriate strength. Specifically, the minimum bending radius of the gas conduit as measured by the method prescribed in JIS B8381 is preferably 1.5 to 5 times, more preferably 1.6 to 4 times, even more preferably 1.7 to 3.8 times, and particularly preferably 1.8 to 3.5 times the outer diameter of the gas conduit 31.

When the minimum bending radius is 1.5 times or more the outer diameter of the gas conduit 31, the strength of the gas conduit 31 tends to be sufficiently high. On the other hand, when the minimum bending radius is 5 times or less the outer diameter of the gas conduit 31, a sufficient flexibility of the tube can be easily secured, so that the handling of the application instrument 10 during operation becomes easy. The same applies to the case where the gas conduit 31 and the electric wiring 32 are put together in one pipe.

The electrical wiring 32 is a wiring for supplying electricity from the power supply unit 20 to the application instrument 10. The electric wiring 32 is connected to the inner electrode 4, the outer electrode 5 and the operation switch 9 of the application instrument 10.

The material of the electric wiring 32 is not particularly limited, and a material used for a known electric wiring can be employed. As examples of the material of the electric wiring 32, a metal lead wire covered with an insulating material and the like can be mentioned.

The controller unit 80 as shown in FIG. 2 is composed of an information processing unit. In other words, the controller unit 80 is equipped with a CPU (central processing unit), a memory and an auxiliary storage device, which are connected by buses. The controller unit 80 operates by executing a program.

The controller unit 80 may, for example, be built into the supply unit 20. The controller unit 80 controls the application instrument 10, and the supply unit 20.

The operation switch 9 for the application instrument 10 is electrically connected to the controller unit 80. When the operation switch 9 is turned on, an electrical signal is sent from the operation switch 9 to the controller unit 80. When the controller unit 80 receives the electrical signal, the controller unit 80 activates the solenoid valve 71 and the flow rate controller 74, and applies a voltage between the inner electrode 4 and the outer electrode 5.

In the present embodiment, when the operation switch 9 is a push button and a user such as a doctor pushes the operation switch 9 once, the controller unit 80 receives the electrical signal described above.

Then, the controller unit 80 opens the solenoid valve 71 for a predetermined period of time to allow the flow rate controller 74 to adjust the flow rate of the plasma generating gas having passed through the solenoid valve 71, and applies a voltage between the inner electrode 4 and the outer electrode 5 for a predetermined period of time. As a result, a predetermined amount of plasma generating gas is supplied to the plasma generating unit 12 from the supply source 70, and the reactive gas is continuously discharged from the application instrument 10 for a predetermined period of time (e.g., several seconds to several tens of seconds, or 30 seconds in the present embodiment).

The wire 90 shown in FIG. 1 is formed by intertwining metal strands. The wire 90 has flexibility. The first end (end (A)) of the wire 90 is fixed to the application instrument 10. The first end of the wire 90 may be detachably attached to the application instrument 10 by way of a metal fitting or the like.

The linear member is not limited to the wire 90, and may be a natural fiber such as cotton and linen, or a rope obtained by twisting at least one fiber selected from synthetic fibers such as a polyester fiber, a nylon fiber, an aramid fiber, an acrylic fiber, a modacryl fiber, and a polyurethane fiber.

An outrigger 101 is fixed to the lower part of the housing 21. In the outrigger 101, a pair of support pieces 103 from the main body 102 can be allowed to protrude or be retracted in a predetermined direction along a horizontal plane. The main body 102 is fixed to the lower surface of the housing 21. The pair of support pieces 103 of the outrigger 101 are supported by the ground surface S such as a floor surface. The ground surface S is a flat surface along a horizontal plane. Since the outrigger 101 is fixed to the housing 21, the housing 21 is less likely to tilt relative to the ground surface S even if the housing 21 receives a load from a certain direction.

A hanger frame 106 is fixed to the upper surface of the housing 21. The housing 21, the outrigger 101, and the hanger frame 106 constitute the supporting part 100.

For example, the hanger frame 106 includes a first vertical frame 107, a horizontal frame 108, a second vertical frame 109, a plurality of reinforcing members 110, and a housing section 111.

The first vertical frame 107 extends upward from the upper surface of the housing 21. The horizontal frame 108 extends along the horizontal plane from the upper end of the first vertical frame 107. The direction in which the horizontal frame 108 extends is preferably a predetermined direction in which the pair of support pieces 103 of the outrigger 101 described above are allowed to protrude and be retracted.

The second vertical frame 109 extends downward from the end of the horizontal frame 108 opposite to the fixed end of the first vertical frame 107.

One of the reinforcing members 110 is fixed to the upper surface of the housing 21 and the first vertical frame 107, thereby reinforcing the portions where the upper surface of the housing 21 and the first vertical frame 107 are fixed.

The other one of the reinforcing members 110 is fixed to the first vertical frame 107 and the horizontal frame 108, thereby reinforcing the portion where the first vertical frame 107 and the horizontal frame 108 are fixed.

The first vertical frame 107, the horizontal frame 108, the second vertical frame 109, and the reinforcing members 110 are formed of, for example, a metal square rod or the like.

The housing section 111 includes a case 114, a roller 115, a ratchet mechanism and an urging member (not shown).

The case 114 is fixed to the lower end of the second vertical frame 109.

The rollers 115 are placed in the case 114 and are supported by the case 114 rotatably around a predetermined axis. The second end (end (B)) of the wire 90 is fixed to the outer peripheral surface of the roller 115, and a part of the wire 90 is wound around the roller 115.

In this embodiment, the end (B) refers to the end of the unwound portion of the wire 90 on the side of the supporting part 100.

The urging member urges the roller 115 around the axis of the roller 115 so that the roller 115 winds in the wire 90. On the other hand, the wire 90 can be unwound from the roller 115 by pulling out the wire 90 from the case 114 against the urging force of the urging member.

The ratchet mechanism has a known configuration and can be switched between a restricted state and a released state. When the ratchet mechanism is in the restricted state, the roller 115 is restricted from winding the wire 90 in the roller 115 and unwinding the wire 90 from the roller 115.

When the ratchet mechanism is in the released state, the roller 115 can wind in the wire 90 or unwind the wire 90 therefrom.

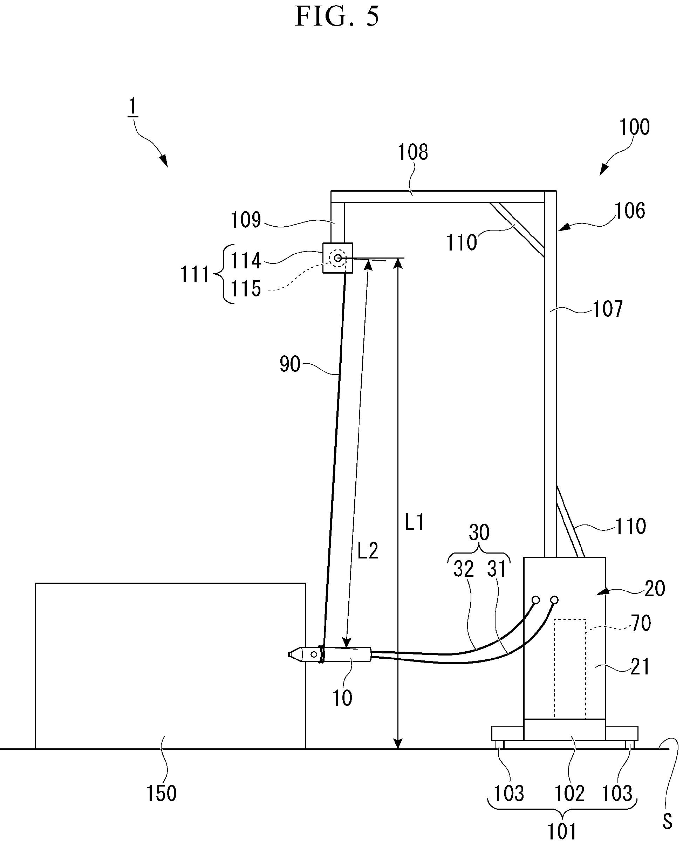

FIG. 5 shows a state in which the wire 90 is entirely unwound from the roller 115.

As can be seen from FIG. 5, the reactive gas application instrument 1 of the present embodiment has a configuration wherein the end (B) of the wire 90 fixed to the supporting part 100 is located at a position higher that the end (A) of the wire 90 fixed to the application instrument 10.

The height L1 of the roller 115 at its position where the end (B) of the wire 90 is fixed from the ground surface S (floor surface or ground) is larger than the length L2 of the wire 90. In this context, the height L1 is not particularly limited, but needs to be at least larger than the height of the application instrument 10 during the use of the reactive gas application apparatus 1, so that the height L1 is preferably 100 cm or more, and more preferably 120 cm or more. The upper limit of the height L1 is also not particularly limited, but is preferably 200 cm or less, and more preferably 180 cm or less, for keeping the entire size of the reactive gas application apparatus 1 at an appropriate level.

In this context, the difference (L1 - L2) between the height L1 of the fixed position of the end (B) of the wire 90 and the length L2 of the wire 90 is preferably 30 cm to 120 cm, and more preferably 50 cm to 100 cm.

The length L3 of the power/gas supply line 30 is not particularly limited, but is preferably 50 cm to 250 cm, and more preferably 100 cm to 200 cm, from the viewpoint of ensuring good operability of the application instrument 10.

Since L1 can be fixed at an arbitrary length by the aforementioned mechanism of the housing section 111, the application instrument 10 can be fixed at the height of the treatment target during the treatment so as to improve the accuracy of aiming the plasma or the like.

Next, a method of using the reactive gas application apparatus 1 having a configuration as described above will be described.
Examples of the target object for the application using the reactive gas application apparatus 1 include cells, biological tissues, and whole bodies of organisms.

Examples of the biological tissues include organs (internal organs, etc.), epithelial tissues covering the body surface and the inner surface of the body cavity, periodontal tissues (gingiva, alveolar bone, periodontal ligament, cementum, etc.), teeth, bones, and the like. Examples of diseases and symptoms that can be treated by application of the reactive gas include diseases in the oral cavity such as gingivitis and periodontal disease, skin wounds and the like.

Examples of the whole bodies of organisms include mammals such as humans, dogs, cats, pigs, etc.; birds; fishes; and the like.

The area to which the labeling composition is applied (application area) is the area to be treated. That is, the application area may be a part or all of the affected area, or a wider area including the affected area.

Hereinbelow, an explanation is made taking, as an example, a case where the target object is a dog and the periodontal tissue of the dog is to be treated.

As shown in FIG. 1, the user P, who is a veterinarian or the like, places a dog D on, for example, a table 150 placed on the ground surface S. During this process, the mouth of the dog D is kept open.

The user P holds the application instrument 10 in his or her hand and presses the operation switch 9 of the application instrument 10. This allows electricity and plasma generating gas to be supplied from the supply unit 20 and the supply source 70 to the application instrument 10 via the power/gas supply line 30.

The plasma generating gas supplied to the application instrument 10 flows into the inner space of the tubular dielectric 3 of the plasma generating unit 12 from the rear end of the tubular dielectric 3. The plasma generating gas is ionized at a position where the inner electrode 4 and the outer electrode 5 face each other, thereby generating plasma.

In the present embodiment, the inner electrode 4 and the outer electrode 5 face each other in a direction orthogonal to the flowing direction of the plasma generating gas. Plasma generated at a position where the outer peripheral surface of the inner electrode 4 and the inner peripheral surface of the outer electrode 5 face each other is allowed to pass through the gas flow path 6 and the reactive gas flow path 7 in this order. In this process, the plasma flows while changing the gas composition, and generates a reactive gas containing reactive species such as radicals.

The application instrument 10 discharges the plasma and the reactive gas (hereinafter, collectively referred to as "plasma or the like") from the reactive gas flow path 7 to the outside.

The user P puts the tip of the application instrument 10 into the mouth of the dog D and points the tip of the application instrument 10 toward the periodontal tissue.
The treatment is performed by applying the plasma or the like to a predetermined part of the periodontal tissue for a certain period of time.

After the treatment of the predetermined part is completed, the plasma or the like is applied to other parts of the periodontal tissue for a certain period of time.

In this way, the entire periodontal tissues in the mouth of the dog D are treated.

During treatment or the like, it is conceivable that the user P holding the application instrument 10 in his or her hand may accidentally drop the application instrument 10 from the hand for some reason. Even in such a case, the application instrument 10 is fixed to the supporting part 100 via the wire 90. Therefore, as shown in FIG. 5, even if the user P releases his or her grip on the application instrument 10, the application instrument 10 is less likely to fall on the ground surface S.

Furthermore, when operating the application instrument 10 without being fixed to the supporting part 100 via the wire 90, it may be difficult to keep holding the application instrument 10 at a desired position. However, in the present embodiment, since the application instrument 10 is fixed to the supporting part 100 via the wire 90, there is an advantage that the application instrument 10 can be easily held at a desired position. This means that it is possible to reliably apply the plasma or the like to a predetermined location of the affected area, which can have a great influence on the therapeutic effect.

As explained above, the plasma application apparatus 1 of the present embodiment can prevent the user P from accidentally dropping the application instrument 10 during use, while ensuring the operability of the application instrument 10.

The supporting part 100 includes the housing section 111. When the wire 90 need not be long in relative terms, the wire 90 can be wound into the housing section 111, whereas when the wire 90 needs to be long in relative terms, the wire 90 can be unwound from the housing section 111. Thus, the length of a part of the wire 90 which is withdrawn out of the housing section 111 can be adjusted.

The height L1 of the roller 115 at its position where the end (B) of the wire 90 is fixed from the ground surface S (floor surface or ground) is larger than the length L2 of the wire 90. Because of this, even when the application instrument 10 accidentally drops from the hand of the user P holding the application instrument 10, it is possible to prevent the dropped application instrument 10 from coming into contact with the ground surface S.

### (Second Embodiment)

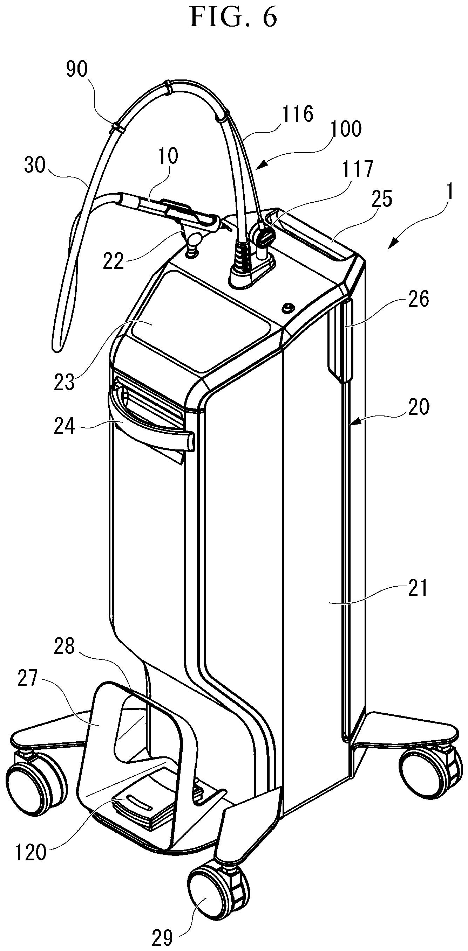

Hereinbelow, the reactive gas application apparatus according to the second embodiment of the present invention is described with reference to FIG. 6. In FIG. 6, the same components as in FIGS. 1 to 5 for explaining the reactive gas application apparatus of the first embodiment are designated by the same reference numerals, and the descriptions thereof are omitted.

In the reactive gas application apparatus 1 of the present embodiment, a power/gas supply line 30 is provided on the upper surface of the supply unit 20 so as to extend upward. A gas conduit and an electric wiring are together housed in the electric power/gas supply line 30.

In the present embodiment, the supporting part 100 is composed of a flexible rod 116 provided on the upper surface of the supply unit 20 so as to extend upward, and a fixing part 117 having a rotatable portion.

The tip of the flexible rod 116 is fixed to the power/gas supply line 30 by a banding band 90 as a connecting part, and the root end of the flexible rod 116 is fixed to the rotatable portion of the fixing part 117. The rotatable portion is preferably configured to be rotatable in the vertical direction and be capable of fixing the flexible rod 116 at a predetermined angle. Examples of a fixing means usable in this instance include a knob screw and the like. The flexible rod 116 may be directly fixed to the upper surface of the supply unit 20 without using the fixing part 117.

The banding band 90 as a connecting part is not particularly limited as long as the power/gas supply line 30 can be fixed to the tip of the flexible rod 116 without excessively squeezing the gas flow path. For example, a known resin banding band or the like can be used. Alternatively, the banding band 90 may be a metal wire or a resin or metallic figure eight ring (which can fix the flexible rod 116 and the power/gas supply line 30 with having the flexible rod 116 and the power/gas supply line 30 respectively passed through the two holes of the figure eight ring).

The flexible rod 116 and the banding band 90 hold the middle portion of the power/gas supply line 30 at a sufficient height, whereby the application instrument 10 can be prevented from falling.

Further, it is preferable that the flexible rod 116 is fixed to the power/gas supply line 30 by the banding band 90 even at a position other than the tip portion. The number of fixed portions other than the tip portion is preferably 3 to 10. When the number of fixed portions is not less than the lower limit value, the downward movement of the application instrument 10 can be more reliably suppressed. Further, even if the fixed portions are provided in a number exceeding the upper limit value, further improvement in the effect cannot be expected, and there would be no potential practical advantage.

When the power/gas supply line 30 is fixed at a position other than the tip of the flexible rod 116, the distance between the adjacent pairs of fixed portions including the tip is preferably 10 cm to 50 cm, more preferably 15 cm to 40 cm, and even more preferably 15 cm to 40 cm.

As the flexible rod 116, one formed of a resin reinforced with fibers such as carbon or glass (FRP or CFRP) can be used.

As can be seen from FIG. 6, the reactive gas application apparatus 1 of the present embodiment of the present embodiment has a configuration wherein the tip of the flexible rod 116 fixed to the power/gas supply line 30 is placed at a position higher than the upper surface of the supply unit 20.

In the present embodiment, the length L2 of the flexible rod 116 is larger than the difference (L3 - L4) between the length L3 of the power/gas supply line 30 and the height L4 of the supply unit 20, that is, it is preferable to satisfy the relationship of L2 > (L3 - L4). For more reliably preventing the user from dropping the application instrument 10 during use while ensuring good operability of the application instrument 10, the difference between the value of L2 and the value of (L3 - L4) is preferably 5 cm to 50 cm, and more preferably 10 cm to 30 cm.

With respect to the flexible rod 116, the thickness or physical properties such as elastic modulus are not particularly limited. For example, appropriate thickness or physical properties may be chosen, which allow the height of the tip of the flexible rod 116 from the upper surface of the supply unit 20 to be maintained at preferably around 10 cm to 100 cm, and more preferably around 20 cm to 50 cm.

In the reactive gas application apparatus 1 of the present embodiment, a holder 22 for holding the application instrument 10 is further provided on the upper surface of the supply unit 20. The holder 22 may also be configured so that the holding angle of the application instrument 10 can be adjusted by providing a rotatable portion as in the case of the fixing part 117. Also in this case, the rotatable portion is preferably configured to allow the holding angle to be fixed at a predetermined angle by a knob screw or the like.

Further, in the reactive gas application apparatus 1 of the present embodiment, the operation panel 23 is provided at the front of the upper surface of the supply unit 20. The operation panel 23 is connected to the control unit 80, and is configured such that the flow rate of the plasma generating gas can be regulated from the operation panel 23 via the control unit 80. Further, the operation panel 23 may be provided with an on/off switch or standby switch for the supply unit 20, a flow rate display unit for plasma generating gas, and the like.

In the reactive gas application apparatus 1 of the present embodiment, a foot switch holder 27 in which the foot switch 120 is installed is provided at the lower part of the front surface of the supply unit 20. The foot switch holder 27 is configured so as to be detachable. The foot switch 120 may be operated with the foot switch holder 27 staying installed at the lower part of the front surface of the supply unit 20, or may be operated after being detached from the foot switch holder 27 and moved to a desired position. The foot switch holder 27 can be moved by grabbing the grip 28 of the foot switch holder 27.

The foot switch 27 can be assigned the same function as the operation switch 9.

As the foot switch 27, a known one can be appropriately adopted, and specific examples thereof include "MKF-MED" manufactured by Steute JAPAN.

In the reactive gas application apparatus 1 of the present embodiment, casters 29 are provided at the bottom of the supply unit 20 so that the reactive gas application apparatus 1 can be easily moved. It is preferable that each of the casters 29 has a lock/unlock mechanism. As the casters 29, known ones can be adopted.

Further, grips 24 and 25 are provided at the front and rear of the supply unit 20, respectively, thereby allowing the reactive gas application 1 to be moved by holding the grips 24 and 25.

In the reactive gas application apparatus 1 of the second embodiment of the present invention as described above, since the power/gas supply line 30 is provided on the upper surface of the supply unit 20 so as to extend upward, it is possible to suppress damage caused by contact of the power/gas supply line 30 with a wall or the like. In addition, the flexible rod 116 enables the achievement of desired effect of the present invention that it is possible to prevent the user from dropping the application instrument accidentally during use, while ensuring the operability of the reactive gas application apparatus 1 as well as maintaining a simple structure and keeping the entire size of the reactive gas apparatus 1 compact. Thus, the reactive gas application apparatus 1 of the second embodiment of the present invention is an apparatus having a configuration suitable for moving or transporting the apparatus.

Further, the supply unit 20 is configured so as to allow access to the inside of the supply unit 20 by removing its back cover holding the recessed handles 26.

Although specific embodiments of the present invention are described above in detail with reference to the drawings, the specific configuration is not limited to these embodiments, and modifications, combinations and deletions of any features may be made as long as such modifications etc. do not deviate from the gist of the present invention.

For example, in the above embodiments, the reactive gas application apparatus 1 may not include the housing section 111, and the second end of the wire 90 may be directly fixed to the lower end of the second vertical frame 109. In this instance, for example, the linear member may be formed of an elastic material such as a spiral spring. The spring constant of the linear member may be set so as to prevent the application instrument 10 from coming into contact with the ground surface S even when the linear member is stretched out under the load of the application instrument 10 and the power/gas supply line 30.

The application instrument 10 may be configured so as to discharge only one of the plasma and the reactive gas.

The first end of the wire 90 may be configured so as to be fixed to at least one of the gas conduit 31 and the electrical wiring 32, which constitute the power/gas supply lines 30. The first end of the wire 90 may be configured so as to be fixed to at least one of the gas conduit 31 and the electrical wiring 32, and to the application instrument 10, respectively.

The plasma application apparatus may be used for humans and non-human animals, or the plasma application apparatus may be used only for non-human animals.

### Industrial Applicability

The plasma application apparatus described above can be suitably used for applications such as treatment of animals and humans, and beauty.

### DESCRIPTION OF THE REFERENCE SIGNS

- 1: Reactive gas application apparatus (plasma application apparatus)
- 10: Application instrument
- 12: Plasma generating unit
- 30: Power/gas supply line
- 90: Connecting part
- 100: Supporting part
- 111: Housing section
- L1: Height from ground surface
- L2: Length of linear member
- S: Ground surface (floor surface or ground)

## Claims

1. A plasma application apparatus comprising:
an application instrument which has a plasma generation unit, and is configured to discharge at least one of plasma generated by the plasma generation unit and a reactive gas generated by the plasma; and
a supply unit for supplying electric power and a plasma generating gas to the application instrument,
a supporting part extending upward from the supply unit,
a power/gas supply line connecting the application instrument to the supply unit, and
a connecting part connecting at least one of the application instrument and the power/gas supply line to the supporting part,
wherein the supporting part and the connecting part maintain the application instrument to stay at a position above a ground surface on which the supply unit is placed.

2. The plasma application apparatus according to claim 1, wherein:
the connecting part is a linear member,
one end (A) of the linear member is fixed to at least one of the application instrument and the power/gas supply line,
the other end (B) of the linear member is fixed to the supporting part, and
the supporting part has a housing section capable of winding the linear member thereinto and unwinding the linear member therefrom.

3. The plasma application apparatus according to claim 1 or 2, wherein the one end (A) of the linear member is fixed to the application instrument,
the other end (B) of the linear member is fixed to the supporting part at its position higher than the one end (A) of the linear member, and
the length of the linear member is smaller than the height of the fixed position of the other end (B) from the ground surface.

4. The plasma application apparatus according to claim 1, wherein the supporting part comprises a flexible rod, wherein:
the connecting part is a banding band that fixes the flexible rod and the power/gas supply line,
the power/gas supply line and the flexible rod are respectively positioned on an upper surface of the supply unit so as to extend upward, and
a middle part of the power/gas supply line is fixed to the flexible rod by the banding band, thereby maintaining the middle part of the power/gas supply line at a position higher than the upper surface of the supply unit.

5. The plasma generating apparatus according to any one of claims 1 to 4, wherein the plasma generating unit generates the plasma by dielectric barrier discharge.

6. The plasma generating apparatus according to any one of claims 1 to 5, wherein the plasma generating unit generates the plasma by using nitrogen gas.
